# EUROPEAN PATENT SPECIFICATION

(11) **EP 3 988 118 B1**
(45) Date of publication and mention of the grant of the patent: **28.01.2026**
(21) Application number: 19933440.0
(22) Date of filing: 24.07.2019
(51) Int. Cl.: A61K 45/00, A61K 31/4439, A61P 39/06

(54) **ANTI-AGING APPLICATION OF JWA GENE AND RELATED COMPOUND**
ANTI-AGING-ANWENDUNG VON JWA-GEN UND ZUGEHÖRIGE VERBINDUNG
APPLICATION ANTI-VIEILLISSEMENT DE GÈNE JWA ET DE COMPOSÉ APPARENTÉ

(30) Priority: 21.06.2019 CN 201910540728
(43) Date of publication of application: 27.04.2022
(73) Proprietor: Simcere Pharmaceutical Co. Ltd., Jiangbei New District, Nanjing Jiangsu 210032 (CN)
(72) Inventor: ZHOU, Jianwei, Nanjing, Jiangsu 210019 (CN); LI, Xiong, Nanjing, Jiangsu 210019 (CN); WEN, Yifan, Nanjing, Jiangsu 210019 (CN); HUANG, Yefei, Nanjing, Jiangsu 210019 (CN); CHEN, Dongyin, Nanjing, Jiangsu 210019 (CN); LIU, Jingwen, Nanjing, Jiangsu 210019 (CN); XU, Jin, Nanjing, Jiangsu 210019 (CN); LI, Aiping, Nanjing, Jiangsu 210019 (CN)
(74) Representative: Novagraaf Group
(86) International application number: PCT/CN2019/097393
(87) International publication number: WO 2020/252845

(56) References cited:
- WO-A1-2014/183491
- CN-A- 1 208 733
- CN-A- 106 632 299
- CN-A- 106 632 299
- ZHAO X ET AL: "JWA antagonizes paraquat-induced neurotoxicity via activation of Nrf2", TOXICOLOGY LETTERS, ELSEVIER, AMSTERDAM, NL, vol. 277, 18 April 2017 (2017-04-18), pages 32 - 40, XP085132993, ISSN: 0378-4274, DOI: 10.1016/J.TOXLET.2017.04.011
- WANG RIHUA ET AL: "Astrocytic JWA deletion exacerbates dopaminergic neurodegeneration by decreasing glutamate transporters in mice", vol. 9, no. 3, 2 March 2018 (2018-03-02), XP093047919, Retrieved from the Internet <URL:https://www.nature.com/articles/s41419-018-0381-8> DOI: 10.1038/s41419-018-0381-8
- WANG SHOUYU ET AL: "JWA regulates XRCC1 and functions as a novel base excision repair protein in oxidative-stress-induced DNA single-strand breaks", NUCLEIC ACIDS RESEARCH, vol. 37, no. 6, 10 February 2009 (2009-02-10), GB, pages 1936 - 1950, XP093047917, ISSN: 0305-1048, DOI: 10.1093/nar/gkp054
- ZHOU YAN ET AL: "JAC4 Protects from X-ray Radiation-Induced Intestinal Injury by JWA-Mediated Anti-Oxidation/Inflammation Signaling", ANTIOXIDANTS, vol. 11, no. 6, 27 May 2022 (2022-05-27), pages 1067, XP093047845, DOI: 10.3390/antiox11061067
- SCOTT MAYNARD: "DNA Damage, DNA Repair, Aging, and Neurodegeneration", COLD SPRING HARBOR PERSPECTIVES IN MEDICINE, vol. 5, no. 10, 1 October 2015 (2015-10-01), US, pages a025130, XP93158544, ISSN: 2157-1422, Retrieved from the Internet <URL:https://www.ncbi.nlm.nih.gov/pmc/articles/PMC4588127/pdf/cshperspectmed-AGE-a025130.pdf> DOI: 10.1101/cshperspect.a025130
- WU, YU: "DNA repair protein JWA involves in aging regulation in mice", CHINA DOCTORAL DISSERTATIONS FULL-TEXT DATABASE, MEDICINE & HYGIENE TECHNOLOGY, vol. 2016, no. 7, 15 July 2016 (2016-07-15), XP055767308
- XU, JIN: "DNA repair and cellular self-regulation based on toxicity pathways", ABSTRACTS OF 2017 (THE 3RD) INTERNATIONAL CONFERENCE ON TOXICITY TESTING ALTERNATIVES & TRANSLATIONAL TOXICOLOGY, 9 July 2017 (2017-07-09), XP055767316, [retrieved on 20210120]
- CHEN, R. QIU, W. LIU, Z. CAO, X. ZHU, T. LI, A. WEI, Q. ZHOU, J.: "Identification of JWA as a novel functional gene responsive to environmental oxidative stress induced by benzo[a]pyrene and hydrogen peroxide", FREE RADICAL BIOLOGY & MEDICINE, ELSEVIER INC, US, vol. 42, no. 11, 24 April 2007 (2007-04-24), US, pages 1704 - 1714, XP022043780, ISSN: 0891-5849, DOI: 10.1016/j.freeradbiomed.2007.02.018

## Description

### TECHNICAL FIELD

The invention relates to the anti-aging uses of a JWA gene and the anti-aging uses of related compounds that can activate the JWA gene, belonging to the field of anti-aging and related diseases drugs.

### BACKGROUND

Aging is a complex biological process. It is usually defined as the progressive loss of physiological integrity with age, which eventually leads to the deterioration of body function and tends to death. It is an irreversible phenomenon. Aging has become an important public health issue of global concern. With the continuous improvement of global medical and health level, human life expectancy is increasing. Therefore, the world is also rapidly entering an aging society. For China alone, the aging population is developing rapidly. It is expected that by the middle of this century, the population over 65 will reach 400 million. Along with the growth of age, incidence rate and prevalence of a series of aging related diseases including cardiovascular diseases including hypertension, atherosclerosis, neurodegenerative diseases such as Parkinson's disease, Alzheimer's disease and chronic metabolic diseases such as type II diabetes, non-alcoholic fatty liver and malignant tumors are also increasing. Therefore, finding or developing effective anti-aging and aging related diseases drugs is an important guarantee to ensure human healthy aging, which has attracted extensive attention of governments and scientists in related fields all over the world.

JWA gene, also known as ARL6IP5 (GenBank AF070523, 1998), is a novel stress-response gene that is initially discovered and cloned from a retinoic acid-induced differentiation model of human bronchial epithelial cells by the inventor Jianwei Zhou and others. JWA gene encodes a cytoskeleton binding protein, has active biological functions, and is moderately expressed in normal tissues and cells. When physical and chemical factors of the environment act on the cells, JWA gene responds quickly, expresses sharply and plays the role of inhibiting oxidative stress and repairing DNA damage, etc. (Nucleic Acids Res 2009, 37(6): 1936-1950; Free Radic Biol Med 2007, 42 (11): 1704-1714).

Wu, "DNA repair protein JWA involves in aging regulation in mice", China Doctoral Dissertations Full-text Database, Medicine & Hygiene Technology, vol. 2016, no. 7, 15 July 2016 (2016-07-15)," and Xu "DNA repair and cellular self-regulation based on toxicity pathways", ABSTRACTS OF 2017 (THE 3RD) INTERNATIONAL CONFERENCE ON TOXICITY TESTING ALTERNATIVES & TRANSLATIONAL TOXICOLOGY, 9 July 2017 (2017-07-09)," also disclose JWA repair protein.

The inventors of this patent found in the preliminary research work that JWA is a novel tumor suppressor gene that controls a plurality of downstream regulating factors to inhibit tumor angiogenesis, metastasis and promote apoptosis. JWA polypeptide (PJP1) is screened and obtained and further used to synthesize a specific polypeptide targeting the integrins (PJP1-RGD). The specific polypeptide causes negligible toxic effects to cells and animals but can significantly inhibit the growth of melanoma tumor and gastric cancer cells, etc. in tumor-bearing mice, and can effectively prevent melanoma metastasis. The research results have been granted the Chinese invention patent, the patent number is CN201310178099.X, and the number of announcement of grant is CN103239710B.

Since then, the inventors of this patent have successively applied for Chinese invention patents with patent numbers CN201610164491.2 and CN201610857409.4 around the research results of JWA gene.

### SUMMARY OF THE INVENTION

The present invention provides:
A compound represented by formula (I) or a pharmaceutically acceptable salt thereof for use in treatment of aging-related diseases, 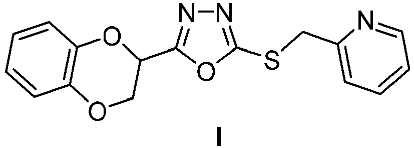 wherein the aging-related diseases are selected from atherosclerosis, skeletal malformations, and type 2 diabetes.

Preferably, the compound is in the form of the compound or a pharmaceutically acceptable salt thereof. The pharmaceutically acceptable salt is prepared by the reaction of the compound with hydrochloric acid, sulfuric acid, benzenesulfonic acid, p-toluenesulfonic acid, phosphoric acid, hydrobromic acid, maleic acid, fumaric acid or malic acid.

Preferably, the compound is an isomer or a homologue of formula I compound.

Preferably, the products are formulations, chips, reagents, kits, healthcare products, medicines or pharmaceutical compositions.

Preferably, the anti-aging comprises delaying aging, prolonging life, improving the appearance of aging-related changes.

In practical research conducted by the inventors, it is found that JWA gene is closely related with aging and therefore can be qualified as a target gene during anti-aging, for treatment of aging-related diseases, anti-oxidation, and maintenance of homeostasis.

Till the disclosure, there have been no small molecule compounds reported that can activate the expression of JWA in vivo to provide anti-aging effects. Therefore, the development of anti-aging drugs based on the JWA as a molecular target has bright application prospect.

### BRIEF DESCRIPTION OF THE DRAWINGS

FIGS. **1-3** show expression levels of JWA protein in the liver and spleen tissues of mice of different ages in accordance with Example **1** of the invention; GAPDH is used as a loading control;
FIGS. **4-6** show body weight curves, the appearance photos and survival curves of JWA^{-/-} mice and wild-type mice in accordance with Example **2** of the invention;
FIGS. **7-10** are images illustrating the phenotype of the aging tissues and organs of JWA^{-/-} mice and wild-type mice in Example **3** of the invention;
FIGS. **11-17** are images illustrating the aging-related phenotype in the cells induced by JWA knockout in accordance with Example **4** of the invention;
FIGS. **18-21** show increases in DNA copy number variation of liver cells induced by JWA knockout in mice in accordance with Example **5** of the invention;
FIGS. **22-28** show decreases in basal metabolism in JWA knockout mice in accordance with Example **6** of the invention;
FIGS. **29-34** show changes in cardiovascular functions in JWA knockout mice in accordance with Example **7** of the invention;
FIG. **35** shows a DNA sequence of 2012 bp upstream of transcriptional start point of the known JWA gene in accordance with Example **8** of the invention;
FIG. **36** is a map of a target pGL3-JWA-Neo vector comprising JWA promoter sequence in accordance with Example **8** of the invention;
FIG. **37** shows Western blot result data demonstrating the expression of JWA protein in HBE cells activated by treating with small molecular compound JAC-4 at different doses in Example **10** of the invention; tubulin is used as a loading control;
FIGS. **38-40** show the results of high-performance liquid chromatography (HPLC) analysis of small molecular compound JAC-4 in test feed in accordance with Example **11** of the invention;
FIGS. **41-42** show body weight curves for the mice (12 months old) fed with test feed (containing 1 g/kg of compound JAC-4) and control feed in accordance with Example **12** of the invention;
FIGS. **43-44** show scoring curves for the aging phenotype of the mice (12 months old) fed with test feed (containing 1 g/kg of compound JAC-4) and control feed in accordance with Example **12** of the invention;
FIG. **45** illustrates a comparison of the appearance of mice (12 months old) before and after being fed with test feed (containing 1 g/kg of compound JAC-4) and control feed in accordance with Example **12** of the invention;
FIG. **46** shows Western blot result data demonstrating the expression of JWA protein in liver and spleen tissues of naturally aging mice (24 months old) fed with test feed (containing 2 g/kg of compound JAC-4) in accordance with Example **13** of the invention; GAPDH is used as a loading control;
FIG. **47** shows HE staining data demonstrating the liver, spleen and kidney of naturally aging mice (24 months old) fed with test feed (containing 2 g/kg of compound JAC-4) and control feed in Example **13** of the invention; wherein the arrow of the liver HE staining data represents inflammatory cell infiltration;
FIG. **48** shows a decrease in the serum glucose and triglyceride of naturally aging mice (24 months old) fed with test feed (containing 2 g/kg of compound JAC-4) in accordance with Example **13** of the invention;
FIG. **49** shows a PAS staining demonstrating the liver of naturally aging mice (24 months old) fed with test feed (containing 2 g/kg of compound JAC-4) and control feed in accordance with Example **13** of the invention; wherein the arrow represents the cells positive for PAS staining indicating glycogen accumulation;
FIG. **50** shows a change in the rate-limiting enzymes related to glucose metabolism in the liver of naturally aging mice (24 months old) fed with test feed (containing 2 g/kg of compound JAC-4) in accordance with Example **13** of the invention;
FIG. **51** shows the Western blot results of the AKT/GSK3β signaling pathway related to glucose metabolism in the liver and the glucose transporter protein Glut2 in liver tissue cell membranes of naturally aging mice (24 months old) fed with test feed (containing 2 g/kg of compound JAC-4) accordance with Example **13** of the invention; GAPDH is used as a loading control;
FIG. **52** shows an increase in SOD in the serum and liver of naturally aging mice (24 months old) fed with test feed (containing 2 g/kg of compound JAC-4) in accordance with Example **13** of the invention.

### DETAILED DESCRIPTION

The present invention is described in further details below with reference to the accompanying drawings and in combination with examples. However, the present invention is not limited to the examples given. Unless otherwise specified, the operations involved in the methods are conventional, and the reagents and materials used are commercially available products. JWA gene has an accession number AF070523 in GenBank, and is represented by the sequence of **SEQ ID NO: 1.** JWA protein is coded by the JWA gene and is represented by the sequence of **SEQ ID NO: 2.**

### Example 1

Expressions of JWA in mouse liver and spleen tissues were decreased with the increase of week age.

The purpose of this example is to detect the expression level of JWA in mouse tissues of different week ages and determine whether the JWA expression was associated with week age.

Mice were euthanized at different week ages (including 16, 19, 48 and 96 weeks old) and liver and spleen tissues were collected, and frozen at -80°C. The total proteins of the liver and spleen tissues were extracted with a mixture of tissue lysate and protease inhibitors, and separated by electrophoresis through 12.5% polyacrylamide gel. The protein bands of 20-25 kDa were cut out of the polyacrylamide gel according to a protein molecular weight marker, and transferred from the polyacrylamide gel onto a polyvinylidene fluoride (PVDF) membrane by constant current wet rotation method. The JWA protein was imprinted with a mouse anti-JWA antibody and a HPR-goat anti-mouse IgG antibody. The expression level of JWA protein was then determined by HPR substrate and a chemiluminescent device.

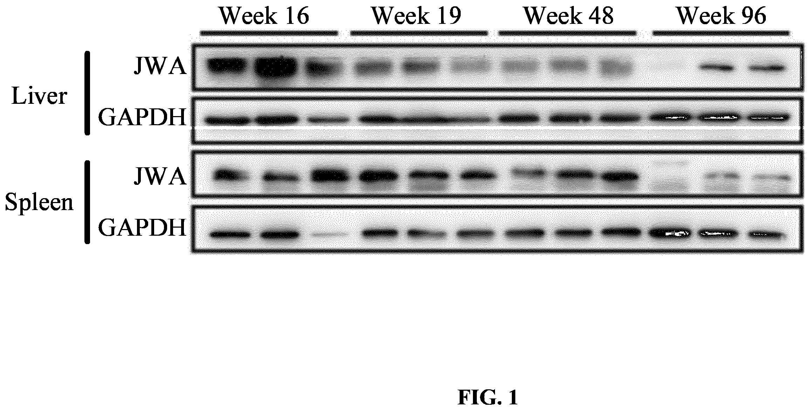

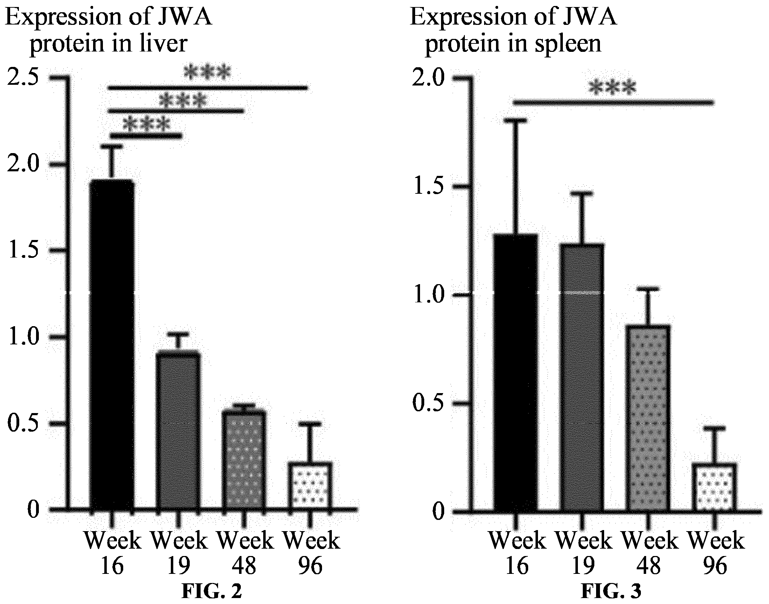

As shown in results of FIGS. **1-3****,** the expression level of JWA protein in the liver and spleen tissues gradually decreased with advancing week age of mice.

### Example 2

Knockout of JWA gene in mice leads to weight loss and life shortening.

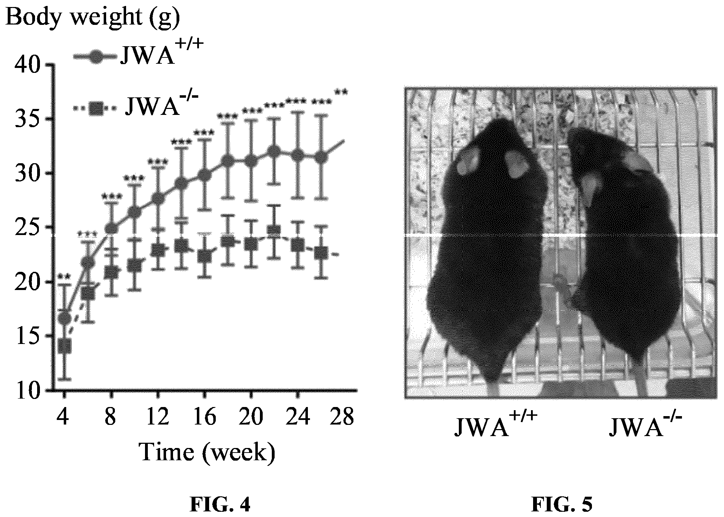

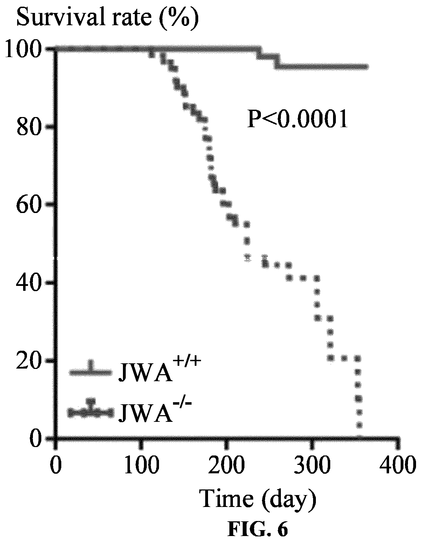

In this example, whole body JWA gene knockout mice constructed by Cre-Loxp system were included in the observation cohort. 927 mice from the cohort were continuously monitored for a prolonged period of time, including 215 homozygous (JWA^{-/-}) mice, 236 wild-type (JWA^{+/+}) mice, and 476 heterozygous (JWA^{+/-}) mice. Obvious ageing phenotype occurred in 90.7% (195/215) of homozygous mice and 4.2% (20/476) heterozygous mice. Referring to FIGS. 4-6, from the 4th week after birth (first detection), the homozygous mice had significant lower body weight and shorter longevity than the wild-type mice. The homozygous mice had a median survival time of 5.8 months (ranging from 1.4-11.8months), while the wild-type mice had a median survival time of 2.5years (30months).

### Example 3

Knockout of JWA gene leads to aging phenotypes in tissues or organs in mice.

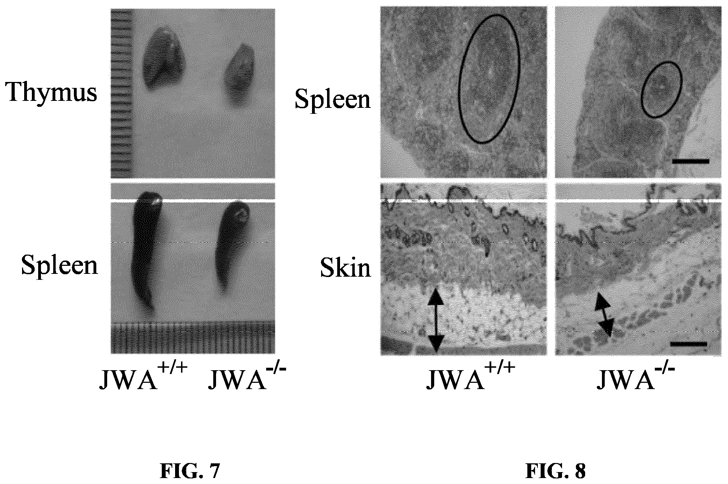

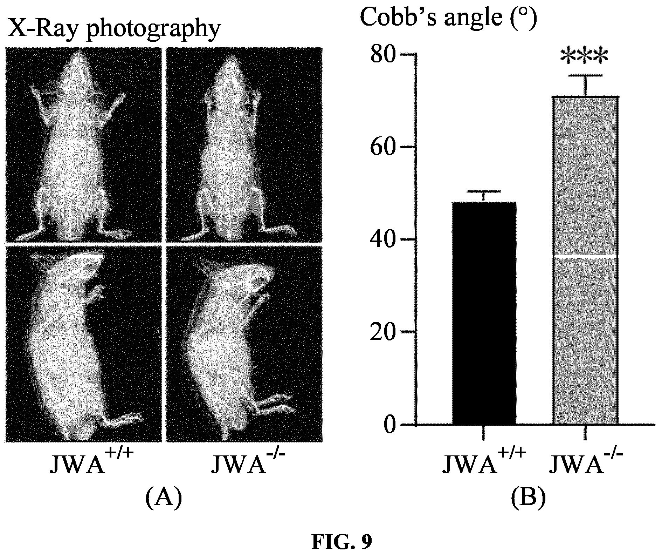

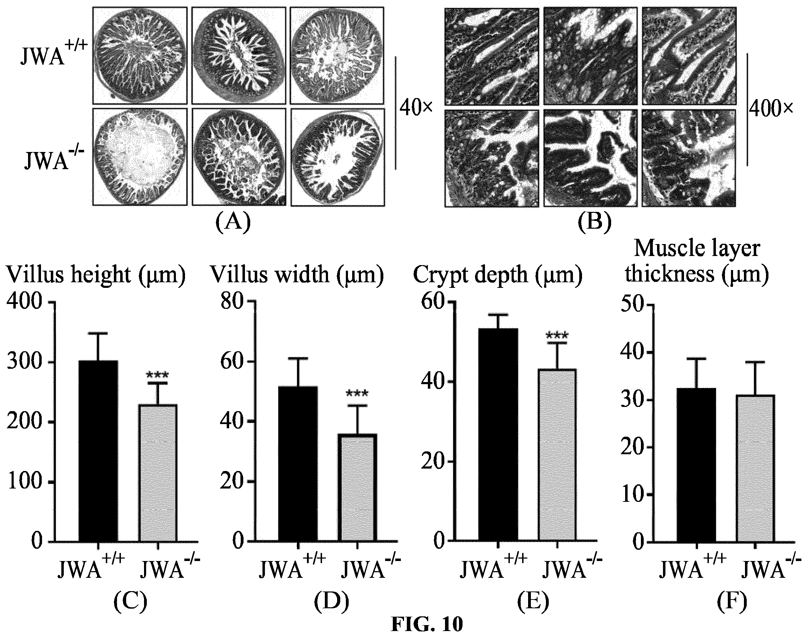

Referring to Example 2, about 90.7% of the homozygous JWA gene knockout mice indicated aging phenotypes including significantly lower body weight and shorter longevity, and showed degeneration of both tissues and organs which results in loss of physical function and death. In this example, gross morphology observation, hematoxylin-eosin (HE) staining for tissue section and X-ray imaging, etc. were conducted in the homozygous JWA knockout mice and wild-type mice at age of 6 months. Referring to FIGS. **7-10****,** the JWA knockout mice produced obvious aging phenotypes in tissues and organs, such as immune organ atrophy, skin atrophy, skeletal deformity, as well as atrophy of villi and crypt of small-intestinal mucosal epithelium.

### Example 4

Knockout of JWA gene leads to cell senescence phenotypes.

Cell senescence is the basis of tissue and organ senescence. In this example, at 6 months of age, the liver tissue of JWA^{-/-} mice and wild-type mice were sectioned and stained with senescence-associated β-galactosidase (SA-β-gal). A Real-Time PCR (RT-PCR) assay was performed to determine the relative telomere length in liver cells. The embryonic fibroblasts (MEFs) were extracted from the JWA^{-/-} mice and wild-type mice, cultured in vitro, treated with hydrogen peroxide (H₂O₂) to induce cell senescence, and stained with SA-β-gal to visualize senescence cells. The cells were detected by flow cytometry to determine the cell division cycle and the proportion of apoptotic cells. Cell count was used to calculate the change of cell doubling rate in each passage, and RT-PCR was used to detect the change of expression of senescence related gene P21 in each passage.

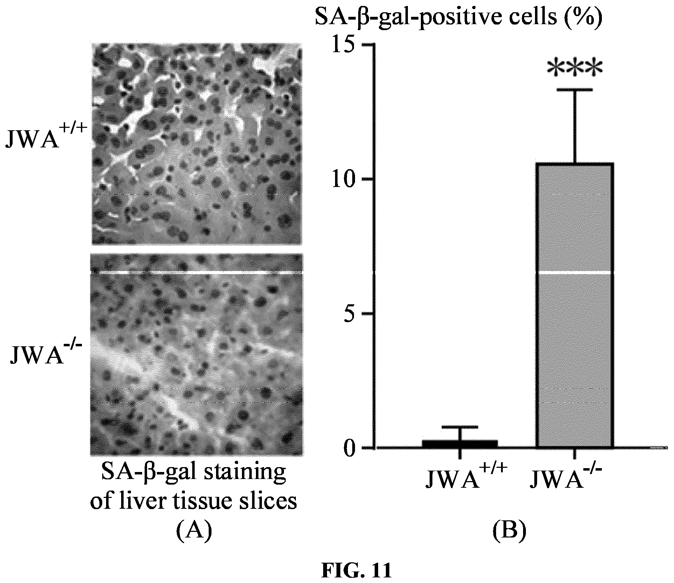

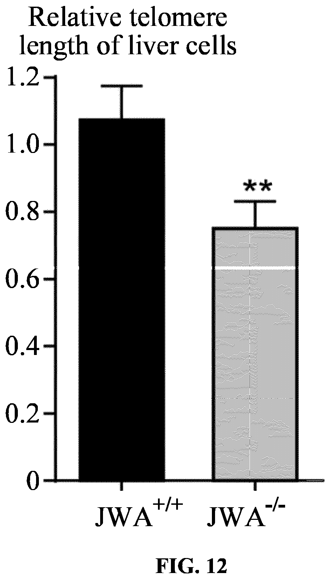

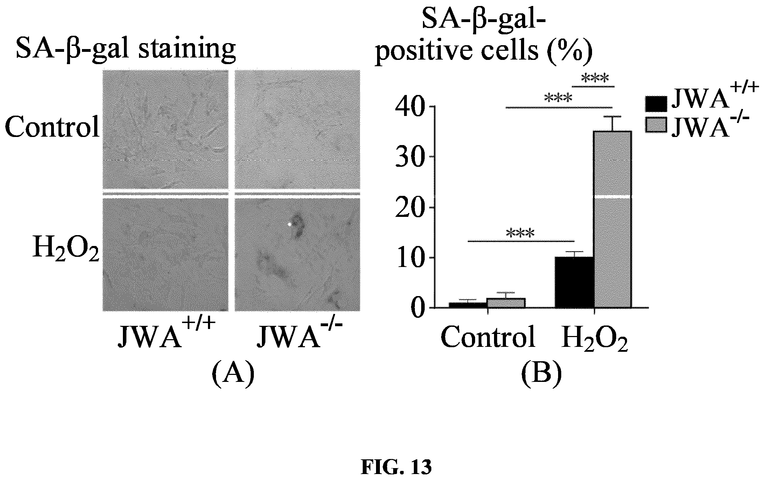

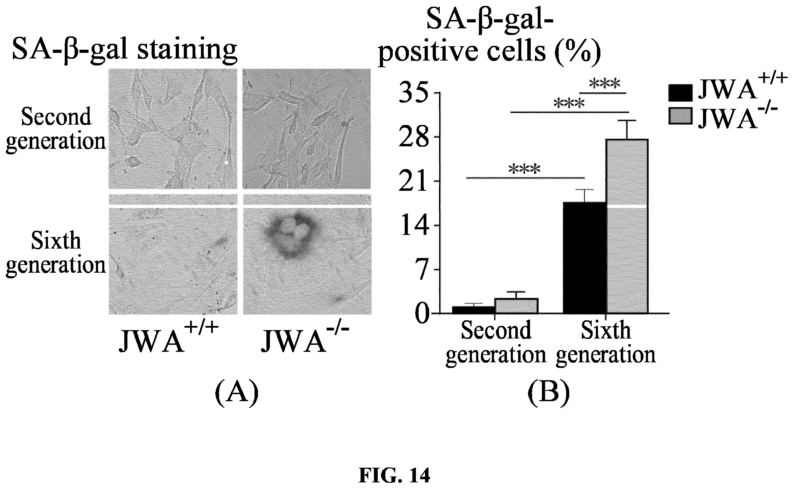

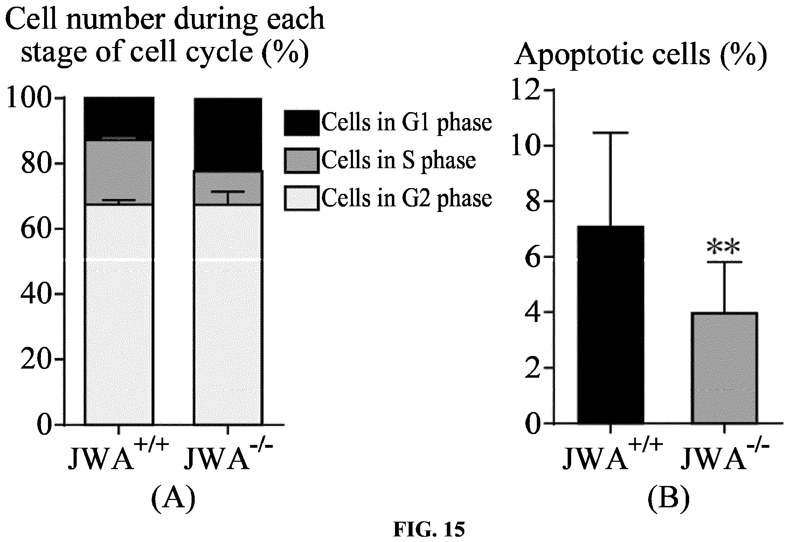

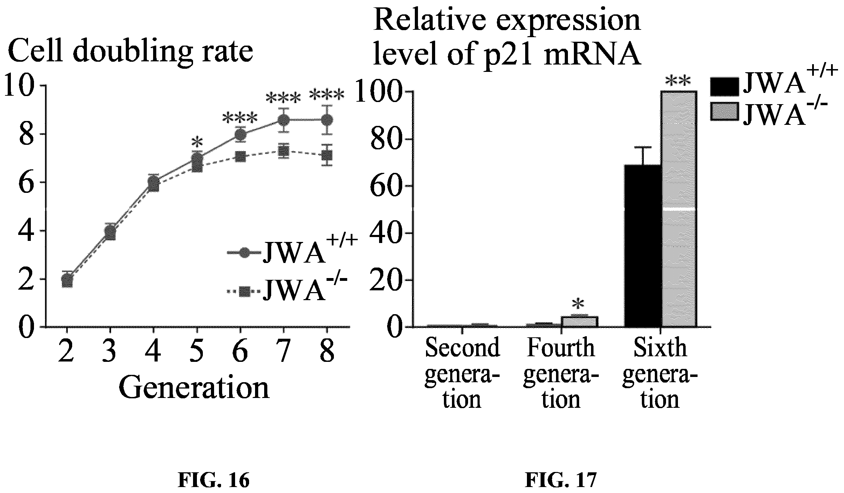

Referring to FIGS. **11-17****,** at 6 months of age, the liver tissue section of JWA^{-/-} mice showed a significantly increase in the number of SA-β-gal positive stained cells compared with the wild-type mice (FIG. 11). The JWA^{-/-} mice had a significantly shorter liver cell telomere length than the wild-type mice (FIG. **12**). When stimulated by H₂O₂, the JWA^{-/-} MEFs showed a marked increase in SA-β-gal positive staining compared with the wild-type MEFs (FIG. **13**). With the generations passing, the JWA^{-/-} MEFs also showed a marked increase in SA-β-gal positive staining compared with the wild-type MEFs (FIG. **14**). The flow cytometry analysis indicated that JWA^{-/-}MEFs showed a significant decrease in levels of cell division (S phase) and apoptotic cells compared with the wild-type MEFs (FIG. **15**), implying that more JWA^{-/-} MEFs were maintained in a state of cell cycle arrest, which meant the cells were aging. With the generations passing, the JWA^{-/-} MEFs had a cell doubling rate of significantly less than the wild-type MEFs (FIG. **16**). The results of RT-PCR also showed that the aging-related gene p21 was expressed at a lower level in the JWA^{-/-} MEFs compared with the wild-type MEFs (FIG. **17**).

### Example 5

Knockout of JWA gene leads to an increase in DNA copy number variation (CNV) in chromosomes in mouse liver cells.

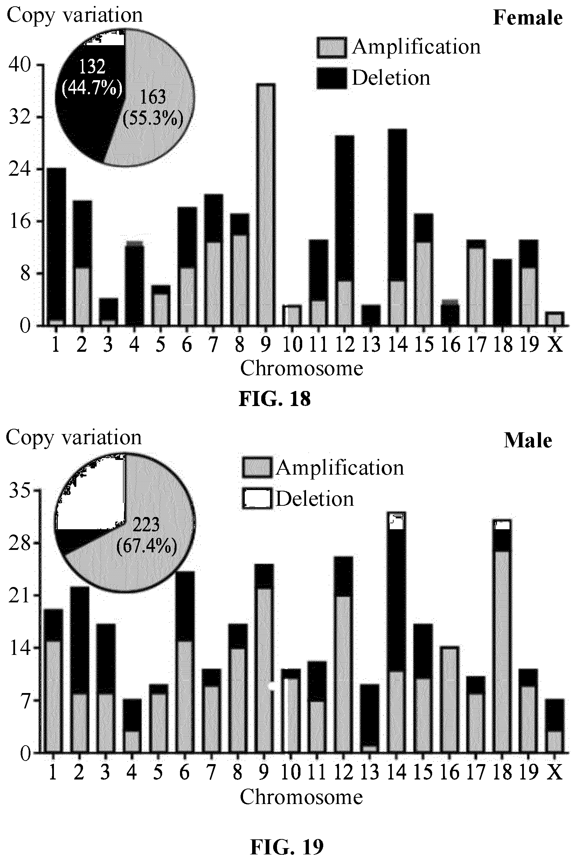

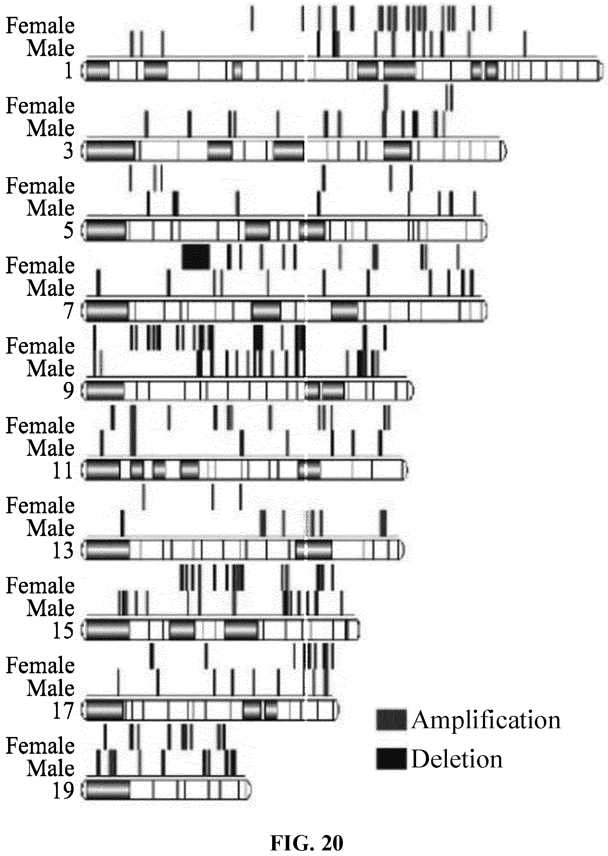

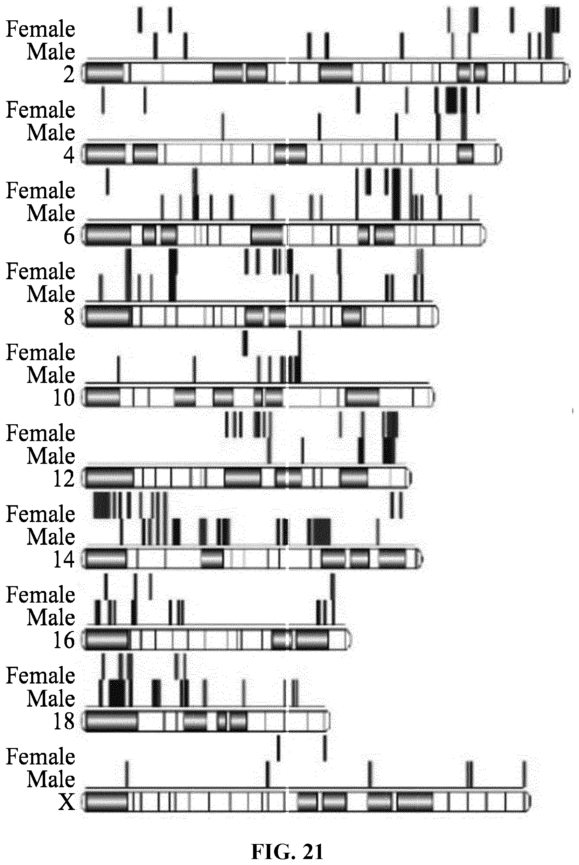

An increase in DNA copy number variation is also defined as one of signs of cellular senescence. In this example, the DNA copy number variation in the chromosomes in the liver cells was detected with comparative genome hybridization microarray. Referring to FIGS. **18-21****,** the DNA copy number variation (i.e. insertion or deletion) in the liver of the JWA^{-/-} mice occurred more frequently than that in the wild-type mice.

### Example 6

Knockout of JWA gene slows down the basal metabolic processes in the mice.

Basal metabolic abnormality is also defined as one of signs of aging. In this example, at 6 months old, the metabolic processes of JWA^{-/-} mice and wild-type mice were measured using the TSE PhenoMaster, a small animal metabolic monitoring system. The main monitoring indictors include daily food intake, daily water intake, O₂ inhalation rate, CO₂ exhalation rate, exercise rate, and thermogenesis rate etc.

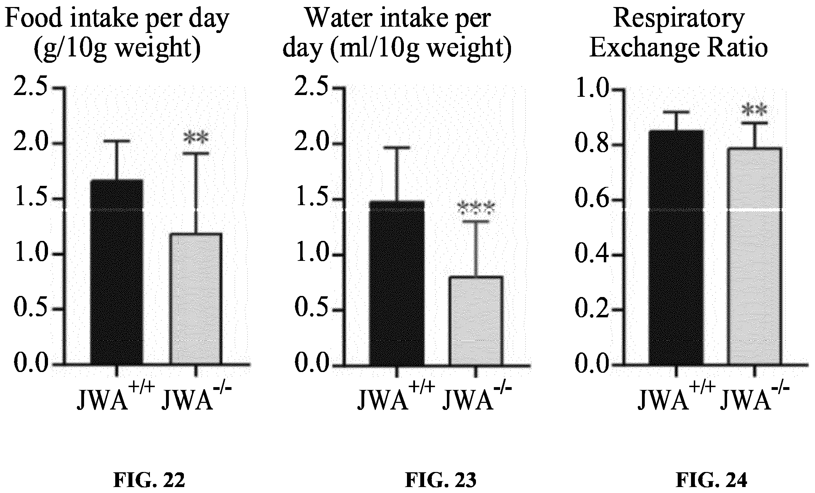

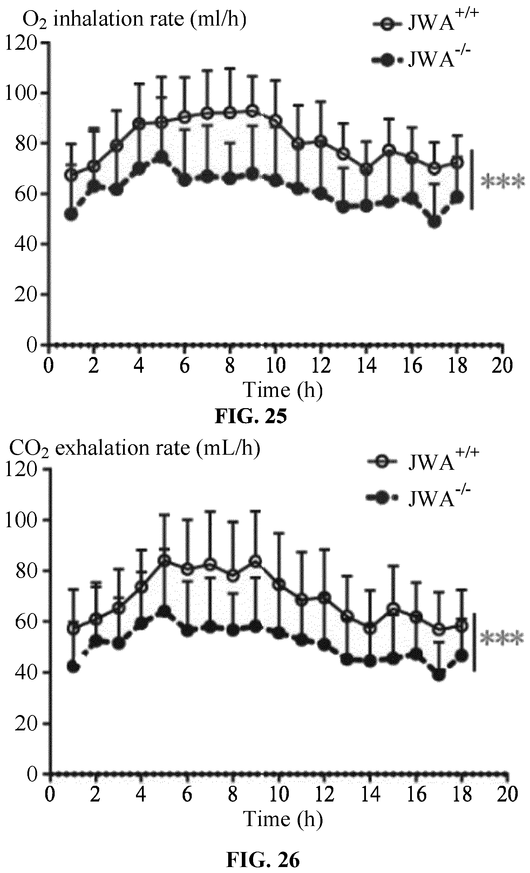

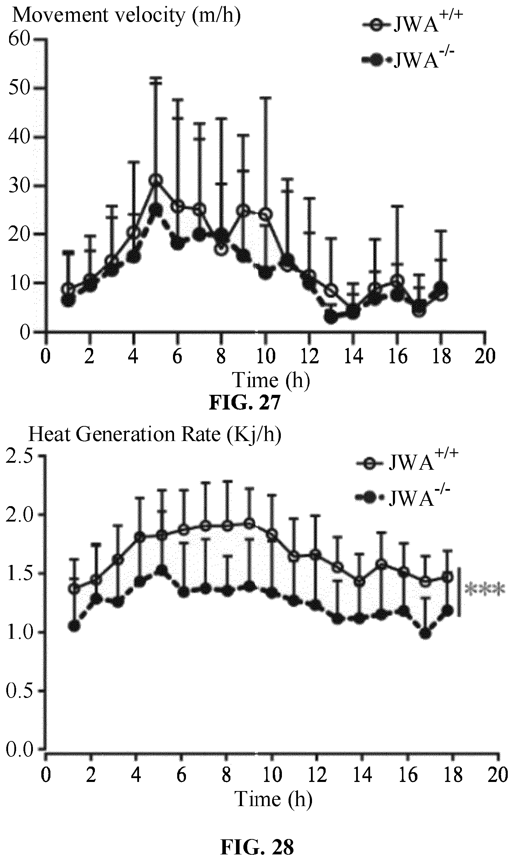

The results shown in FIGS. **22-28** indicate that the JWA^{-/-} mice had a significantly lower basal metabolic rate than the wild-type mice, which is manifested in the decrease in the daily food intake (FIG. **22**) and daily water intake (FIG. **23**). The JWA^{-/-} mice had a decreased respiration rate (FIG. **24**), wherein the O₂ inhalation rate (FIG. **25**) and CO₂ exhalation rate (FIG. **26**) both decreased. Although no significant change in the exercise rate (FIG. **27**), the heat production rate of the JWA^{-/-}mice was significantly lower than that of the wild-type mice (FIG. **28**).

### Example 7

Knockout of JWA gene induced changes in the cardiovascular function of mice.

Aging is one of the major risk factors for chronic cardiovascular diseases. In this example, at 6 months old, a BP-2000 non-invasive blood pressure monitor of rats and mice was used to detect indicators of blood pressure and pulse rate, etc. in mice. Heart functions of mice were detected by Vevo 2100 high frequency color ultrasound system, a small animal imaging system.

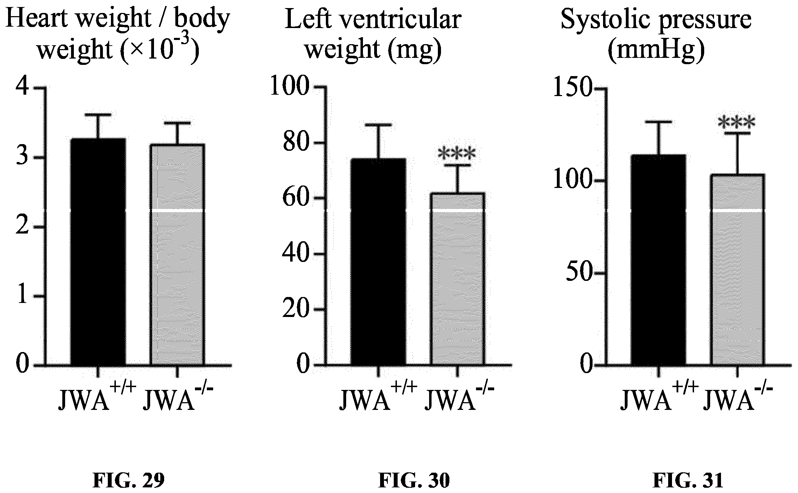

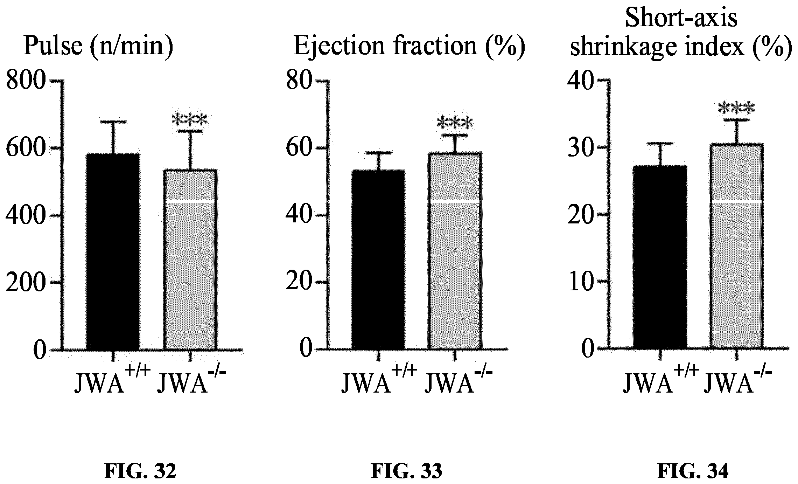

Referring to FIGS. **29-34****,** the JWA^{-/-} mice showed no significant difference in the ratio of heart-weight to body weight (FIG. **29**), but a significant decrease in left ventricular mass compared with the wild-type mice (FIG. **30**), caused reduced systolic pressure (FIG. **31**) and pulse rate (FIG. **32**), and led to compensatory enhancement of the left ventricular ejection fraction (FIG. **33**) and short-axis contraction index (FIG. **34**) in JWA^{-/-} mice.

### Example 8

Construction of reporter gene plasmid of environmental response gene JWA promoter with neomycin resistance.

The object of this example is to construct the reporter vector plasmid containing JWA gene promoter which is further used for screening compounds that can activate the expression of the JWA gene effectively.

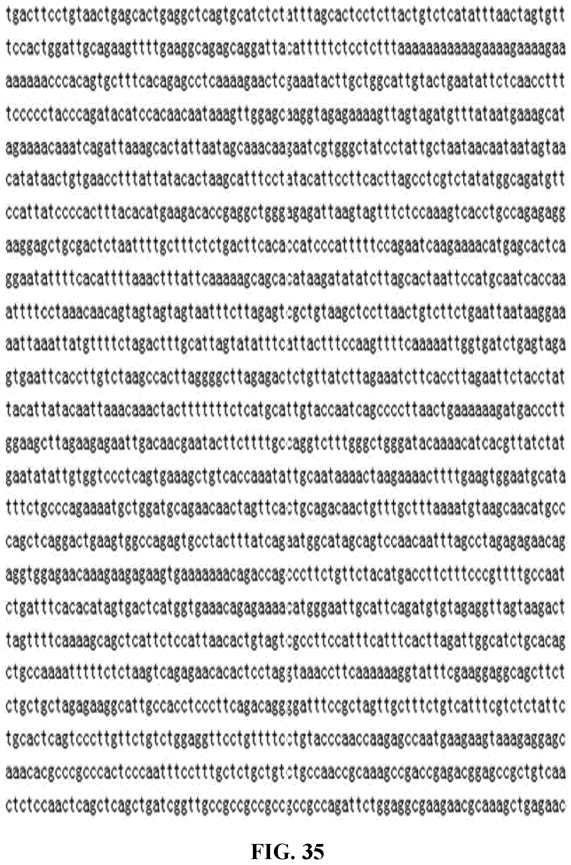

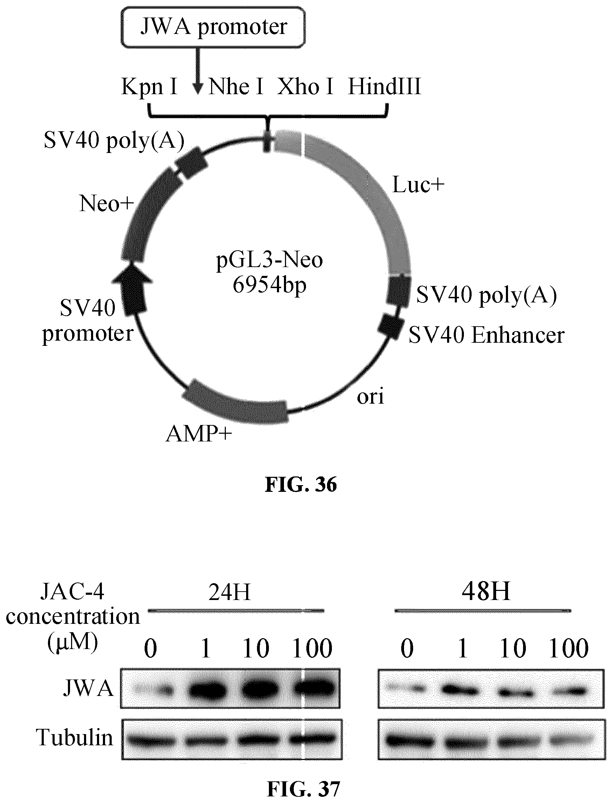

2012 bp upstream of the JWA transcription start site was amplified by RT-PCR according to the human-derived JWA promoter sequence (FIG. **35**), and inserted into a pGL3-Neo vector by using KpnI and NheI restriction enzyme sites, thereby yielding a recombinant vector (FIG. **36**) containing the promoter sequence. The recombinant vector was transferred into E. coli. (DH5α). Positive clones were further sequenced to verify accuracy, thereby obtaining a neomycin-resistant reporter plasmid containing the JWA promoter successfully. The obtained plasmid and corresponding strains were stored at -70°C.

### Example 9

Screening of regulatory molecules specific to JWA.

The object of this example is that the JWA promoter reporter gene assay was performed to screen the compounds that capable of significantly activating the expression of JWA.

Cells were cultured at a density of 3000 cells per well in a 384-well plate and transfected after adhere to the wall. 6.25 µL of serum and antibiotic-free DMEM was added to a mixture of plasmid DNA (0.04 µg) and renilla luciferase phRL-tk (0.01 µg) in the ratio of 4:1, mixed and incubated at room temperature for 5 minutes, thereby obtaining solution A. 1 µL of lipofe2000 transfection reagent was added to 6.25 µL of serum and antibiotic-free DMEM, mixed and incubated at room temperature for 5 minutes, thereby obtaining solution B. The solution A was mixed with the solution B (B was added into A) and incubated at room temperature for 20 minutes. The culture medium in the 384-well plate was discarded and the cells were washed 2-3 times with PBS. 25 µL of serum containing and antibiotic-free DMEM was added. The A and B mixture solution was added when incubation was finished, shaken and placed inside a cell culture incubator for 24 hours. The culture medium was then discarded and replaced with complete DMEM. The cells were treated for 24 hours with a certain concentration of different compounds.

After incubation, the 384-well plate was taken out, allowing the reagents and cells to equilibrate to room temperature of 18-22°C. 60µL of culture medium was aspirated from each well (the remaining culture medium per well was 20 µL), added an equal volume (20 µL) of a detection reagent, and incubated on a shaker in dark at room temperature for 30 minutes. The luciferase activity was detected by using a Dual-Luciferase Reporter Assay kit of Promega company and measured by a chemiluminescence analyzer (3010C chemiluminescence analyzer). All experiments were performed with triplicate cultures.

The fluorescence value of greater than x+3S was used to identify compounds that capable of significantly activating the expression of JWA. The compound of the formula I, which is named JAC-4, showed a good dose-effect relationship and further described by the following examples.

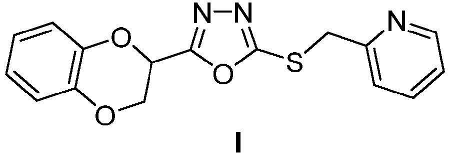

### Example 10

Effects of the small molecule compound JAC-4 on the expression level of JWA protein in human bronchial epithelial (HBE) cells.

The purpose of this example is to verify the accuracy of the screening results through the cell model, and to confirm that JAC-4 does indeed increase the expression of JWA protein.

The HBE cells in the logarithmic growth phase were conventional digested, plated at a density of 5 × 10⁵/cell onto a 60 mm cell culture dish, and treated with different doses of the small molecule compound JAC-4 for 24 hours or 48 hours. 0.18 mL of RIPA(containing 0.5% PMSF) cell lysate for protein extraction was added, and centrifuged at 12,000 ×g for 15 minutes. The supernatant was collected and the protein concentration was measured. The proteins were heated and separated by electrophoresis through 12.5% polyacrylamide gel. 70 µg of the proteins was loaded into each well and separated by electrophoresis at 60 V for 30 minutes and 90 V for 1-1.5 hours. The proteins were then transferred from the gel to a PVDF membrane using a semi-dry transfer apparatus. The PVDF membrane was then blocked with 5% skim milk, incubated at room temperature for 1-2 hours, and washed with TBST (containing 0.1% Tween 20). The washing step was repeated 3 times for 5 minutes each time. The blocked membrane was incubated overnight with corresponding antibody at 4°C, and washed with TBS (containing 0.1% Tween 20) next day. The washing step was repeated 3 times for 5 minutes each time. The blocked membrane was incubated at room temperature for 1-2 hours with secondary antibody, and then washed with TBST (containing 0.1% Tween 20). The washing step was repeated 8 times for 5 minutes each time. ECL luminescent liquid was added onto the membrane to enhance chemiluminescent.

Referring to FIG. **37****,** the expression of JWA protein was effectively enhanced by treating with JAC-4 at concentrations of 1, 10, and 100 µmol/L for 24 hours and 48 hours respectively.

### Example 11

Preparation of experimental animal feed comprising the small molecule compound JAC-4.

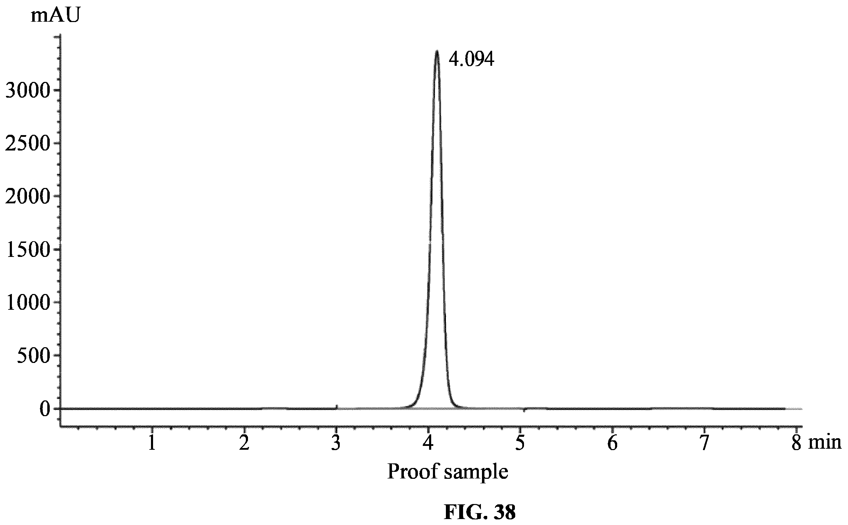

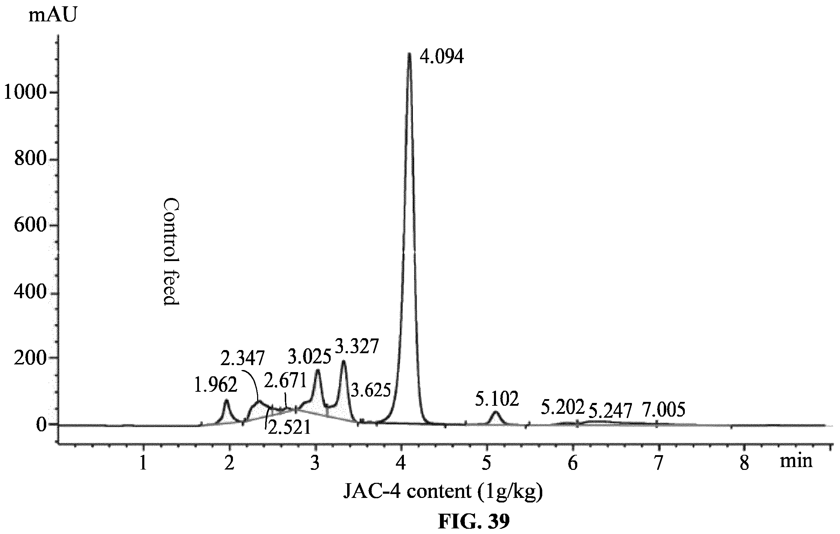

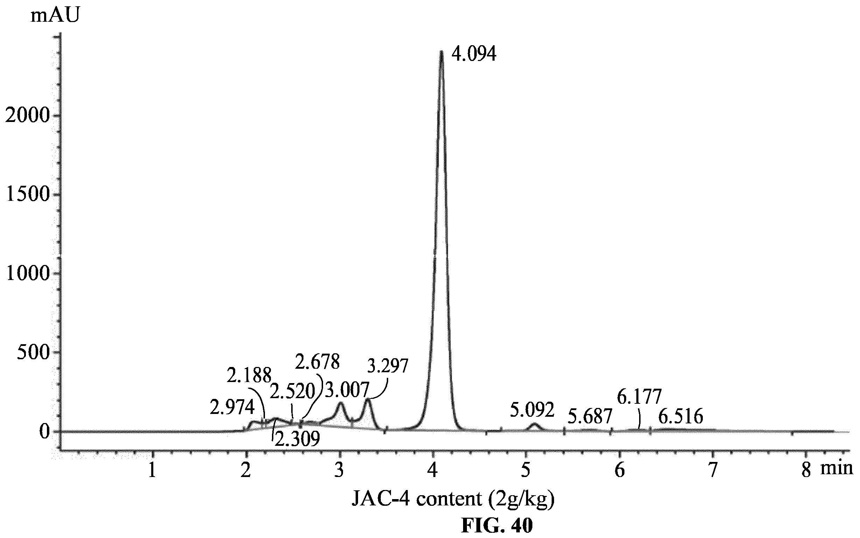

The compound JAC-4 (purity>99%) was mixed with an equal amount of standard mouse feed. Specifically, 1 g of compound JAC-4 was thoroughly mixed with 1 g of basic feed materials, 2 g of the mixture was thoroughly mixed with 2 g of basic feed materials, 4 g of the mixture was thoroughly mixed with 4 g of basic feed materials, and so on, thereby obtaining two test feeds respectively containing 1 g/kg and 2 g/kg of the small molecule compound JAC-4. According to the standard operating procedures of feed company, the test feeds were processed into pellets, dried, vacuum packaged, and sterilized by a Co60 source irradiation. The test feed processing was entrusted to a professional company. To confirm that the compound JAC-4 was evenly distributed in the mixed feeds and not damaged in the structure during processing according to design requirements, the sterilized feeds were randomly sampled and fully extracted with organic solvents. The content of the compound JAC-4 was then measured by High Performance Liquid Chromatography and it was confirmed that the compound JAC-4 was not damaged and the content thereof met the requirements (FIGS. **38-40**).

### Example 12

Feeding 12-month-old mice with the test feed containing 1 g/kg of the compound JAC-4.

12-month-old mice were housed in specific pathogen free (SPF) conditions at an ambient temperature of 22±2°C and a relative humidity of 40%-60%, in day/night cycles of 12 h/12 h simulated by lighting. Mice were fed with sterile acidified water, control feeds (without compound JAC-4) or test feeds (with compound JAC-4). Based on the average body weight of 20 g per mouse and the daily intake of feed for each mouse was 10% of body weight (2g), the daily intake of JAC-4 is about 100 mg/kg of body weight. The mice were weighed every two weeks from the start of feeding and scored on the aging phenotypes every two weeks from the sixth week. The higher the score, the more obvious the aging phenotypes. The mice were photographed monthly and changes in appearance were recorded. The animal experiments involved in this example meet the ethical requirements of experimental animals.

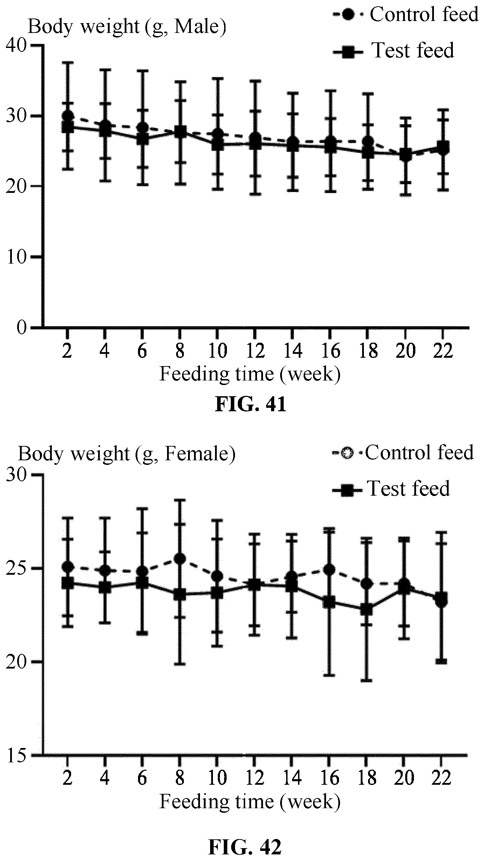

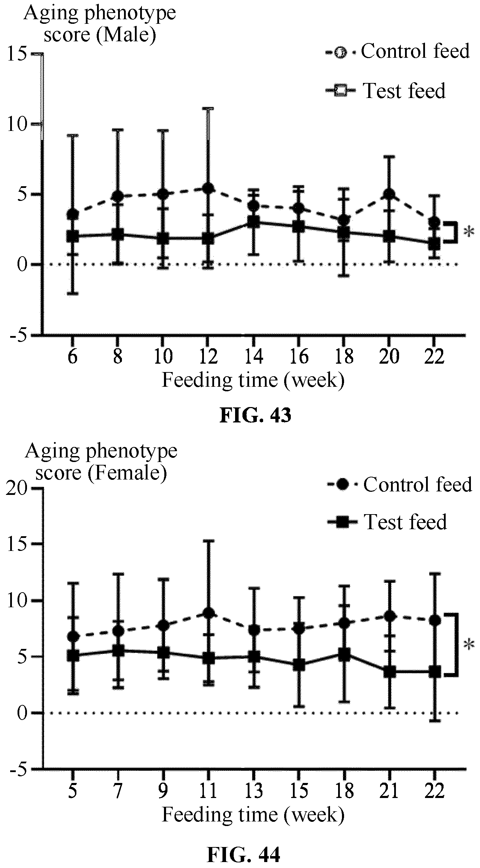

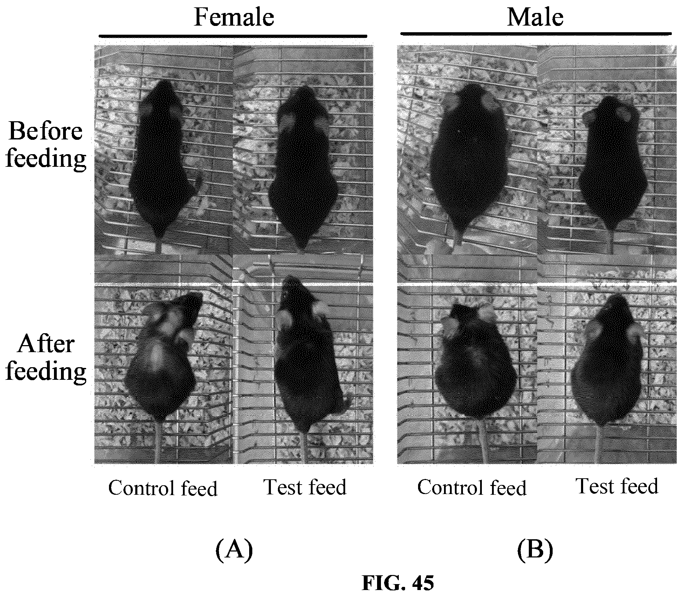

Referring to FIGS. **41** and **42****,** at the 22^{nd} week, the mice fed with the test feeds showed a slightly decrease in body weight compared with the mice fed with the control feeds, but no statistical difference. Referring to FIGS. **43** and **44****,** the mice fed with the test feeds showed a significant decrease in the score of aging appearance compared with the mice fed with the control feeds. Referring to FIG. **45****,** the mice fed with the control feeds showed a significant hair loss. The above results indicate that JAC-4 provides a significant anti-aging effect.

### Example 13

Feeding 24-month-old natural aging mice with the test feed containing 2g/kg of the compound JAC-4.

Mice were housed in the same conditions as Example 12. Based on the average body weight of 20 g per mouse and the daily intake of feed for each mouse was 2 g, the daily intake of the small molecule JAC4 is about 200 mg/kg of body weight. After feeding for 10 weeks, the mice were anesthetized. The whole blood was collected, allowed to stand for 30 min at room temperature, and centrifuged at 3000 rpm for 15 min. The supernatant serum, was separated and analyzed by Hitachi 7100 automatic biochemical analyzer. Liver, spleen and kidney tissues of the mice were isolated on ice, fixed with 4% paraformaldehyde, or frozen at -80°C. The fixed tissues were processed into a paraffin section or frozen section with a thickness of 5 µm, stained with HE to visualize the morphological and pathological changes in the liver, spleen and kidney. The liver section was stained with oil red O for fat deposition and stained with periodic acid-Schiff (PAS) for glycogen. Total proteins and cell membrane proteins were extracted from the liver tissue frozen at -80°C, and total proteins were extracted from the spleen tissue, respectively. Western blot was performed to detect the expression of JWA protein in liver and spleen tissues, the expression of p-AKT, AKT, p-GSK3β and GSK3β proteins in liver tissues, and the expression of glucose transporter (Glut 2) in cell membrane proteins of liver tissues. A biochemical kit (purchased from Nanjing Jiancheng Bioengineering Institute) was used to detect the levels of Superoxide dismutase (SOD), as well as the main rate-limiting enzymes of glucose and lipid metabolism in liver tissues. The animal experiments involved in this example meet the ethical requirements of experimental animals.

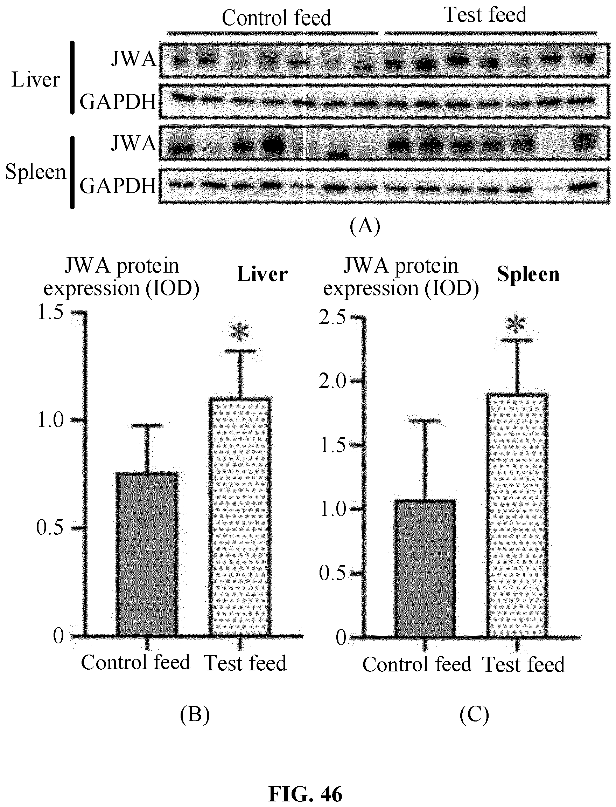

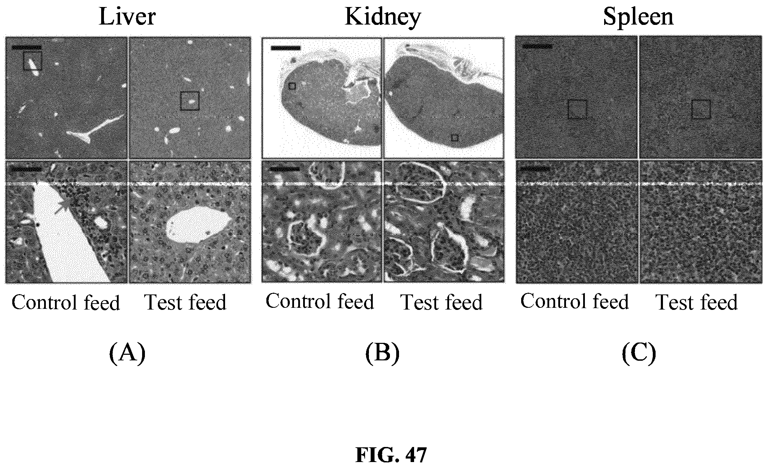

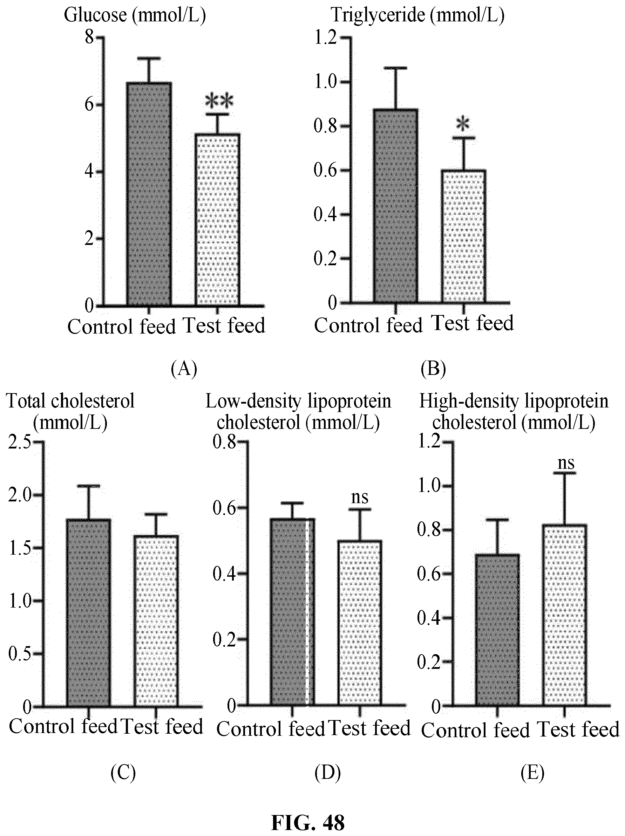

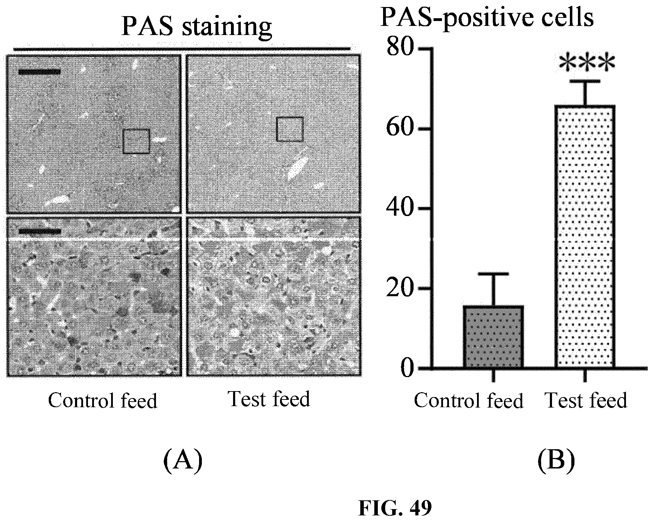

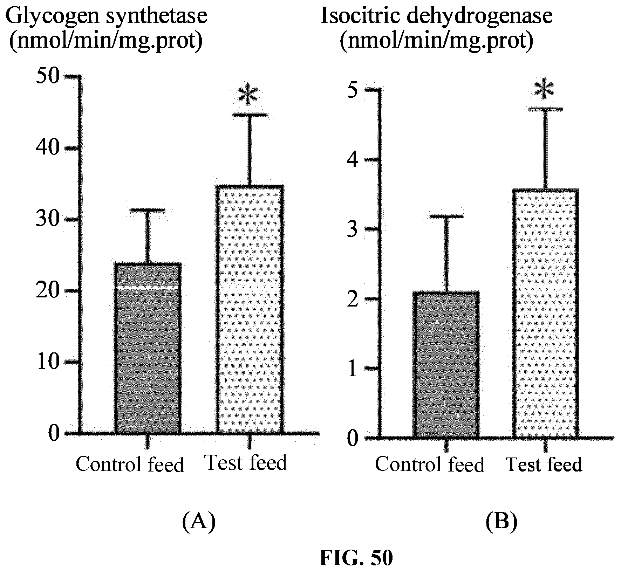

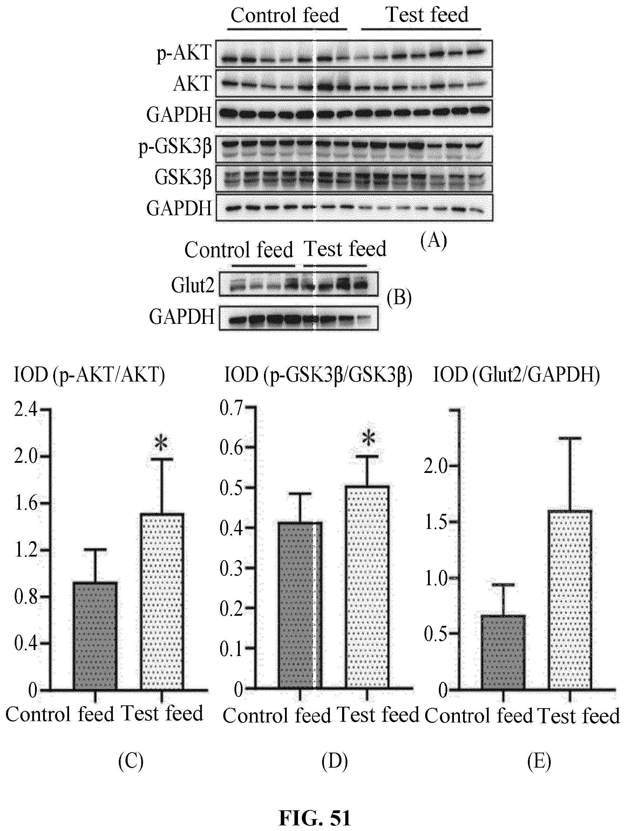

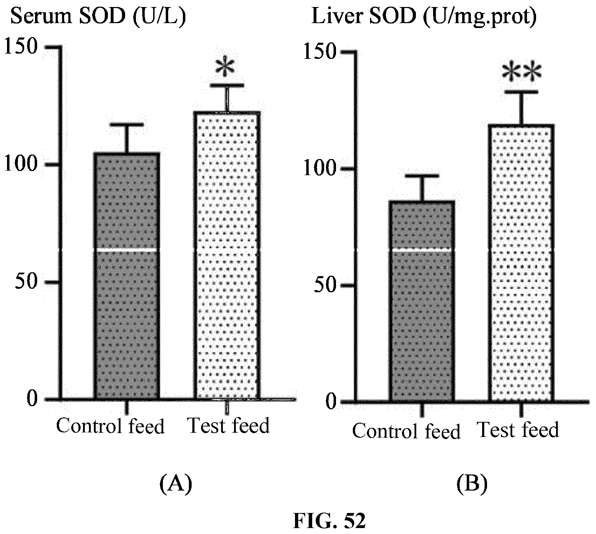

Referring to FIGS. **46-52****,** after feeding the test feed containing compound JAC-4, the expression level of JWA protein was increased significantly in the liver and spleen tissues of aging mice. The compound JAC-4 did not produce abnormal changes in the morphology of mouse liver, kidney and spleen, and significantly reduced age-related inflammatory cell infiltration around the central vein of the liver. JAC-4 also significantly reduced the levels of the serum glucose and triglycerides in naturally aging mice and increased the glycogen synthesis in the liver. Experimental results further indicated that the compound JAC-4 increased the rate-limiting enzyme related to glucose decomposition and the glycogen synthase in the liver, increased expression of cell membrane glucose transporter protein Glut 2, activated AKT phosphorylation, and inhibited GSK3β to promote glycogen synthesis.

The above examples show that the compound JAC-4 activated JWA gene expression to accomplish the function such as delaying aging and maintaining homeostasis.

## Claims

1. A compound represented by formula (I) or a pharmaceutically acceptable salt thereof for use in treatment of aging-related diseases, 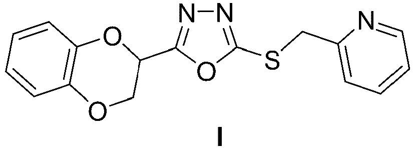 wherein the aging-related diseases are selected from atherosclerosis, skeletal malformations, and type 2 diabetes.

2. The compound or the pharmaceutically acceptable salt thereof for use according to claim 1, wherein the pharmaceutically acceptable salt is prepared by the reaction of the compound with hydrochloric acid, sulfuric acid, benzenesulfonic acid, p-toluenesulfonic acid, phosphoric acid, hydrobromic acid, maleic acid, fumaric acid or malic acid.

3. The compound or the pharmaceutically acceptable salt thereof for use according to 1 or 2, wherein the products are formulations, chips, reagents, kits, healthcare products, medicines or pharmaceutical compositions.

## Patentansprüche

1. Verbindung, die durch die Formel (I) dargestellt wird, oder ein pharmazeutisch verträgliches Salz davon zur Verwendung bei einer Behandlung von alterungsbedingten Krankheiten, 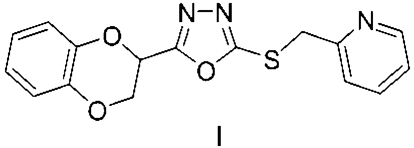 wobei die alterungsbedingten Krankheiten aus Atherosklerose, Skelettfehlbildungen und Typ-2-Diabetes ausgewählt sind.

2. Verbindung oder pharmazeutisch verträgliches Salz davon zur Verwendung nach Anspruch 1, wobei das pharmazeutisch verträgliche Salz durch die Reaktion der Verbindung mit Salzsäure, Schwefelsäure, Benzolsulfonsäure, p-Toluolsulfonsäure, Phosphorsäure, Bromwasserstoffsäure, Maleinsäure, Fumarsäure oder Äpfelsäure hergestellt wird.

3. Verbindung oder pharmazeutisch verträgliches Salz davon zur Verwendung nach 1 oder 2, wobei die Produkte Formulierungen, Chips, Reagenzien, Kits, Gesundheitsprodukte, Arzneimittel oder pharmazeutische Zusammensetzungen sind.

## Revendications

1. Composé représenté par la formule (I) ou sel pharmaceutiquement acceptable de celui-ci pour une utilisation dans le traitement des maladies liées au vieillissement, 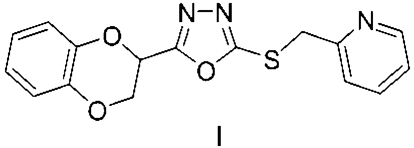 dans lequel les maladies liées au vieillissement sont choisies parmi l'athérosclérose, les malformations du squelette et le diabète de type 2.

2. Composé ou sel pharmaceutiquement acceptable de celui-ci pour une utilisation selon la revendication 1, dans lequel le sel pharmaceutiquement acceptable est préparé par la réaction du composé avec l'acide chlorhydrique, l'acide sulfurique, l'acide benzènesulfonique,
l'acide p-toluènesulfonique, l'acide phosphorique, l'acide bromhydrique, l'acide maléique, l'acide fumarique ou l'acide malique.

3. Composé ou sel pharmaceutiquement acceptable de celui-ci pour une utilisation selon la revendication 1 ou 2, dans lequel les produits sont des formulations, des puces, des réactifs, des kits, des produits de santé, des médicaments ou des compositions pharmaceutiques.
